# EUROPÄISCHE PATENTSCHRIFT

(11) **EP 4 174 160 B1**
(45) Veröffentlichungstag und Bekanntmachung des Hinweises auf die Patenterteilung: **12.03.2025**
(21) Anmeldenummer: 21204840.9
(22) Anmeldetag: 26.10.2021
(51) Int. Cl.: C12M 1/00

(54) **VERFAHREN UND VORRICHTUNG ZUR TRENNUNG EINES GASGEMISCHES**
METHOD AND DEVICE FOR SEPARATING A GAS MIXTURE
PROCÉDÉ ET DISPOSITIF DE SÉPARATION D'UN MÉLANGE GAZEUX

(43) Veröffentlichungstag der Anmeldung: 03.05.2023
(73) Patentinhaber: Alpha Engineering Services GmbH, 67227 Frankenthal (DE)
(72) Erfinder: PLONUS, Frank, 75223 Niefern-Öschelbronn (DE); HEUTE, Steffen, 68519 Viernheim (DE); KOLB, Leon, 68167 Mannheim (DE)
(74) Vertreter: Andrejewski - Honke Patent- und Rechtsanwälte Partnerschaft mbB

(56) Entgegenhaltungen:
- EP-A1- 3 628 390
- WO-A1-2013/087046
- DE-A1- 102008 025 971
- DE-A1- 102008 060 310
- DE-A1- 102012 101 991
- DE-B3- 102005 051 952
- US-A1- 2018 112 142
- LAURA ANNAMARIA PELLEGRINI ET AL: "Biogas to liquefied biomethane via cryogenic upgrading technologies", RENEWABLE ENERGY, vol. 124, 31 August 2018 (2018-08-31), GB, pages 75 - 83, XP055711440, ISSN: 0960-1481, DOI: 10.1016/j.renene.2017.08.007
- CH ET AL: "Climate Friendly Farming Purification Technologies for Biogas Generated by Anaerobic Digestion", CSANR RESEARCH REPORT, 31 January 2010 (2010-01-31), pages 1, XP055122475, Retrieved from the Internet <URL:http://csanr.wsu.edu/wp-content/uploads/2013/02/CSANR2010-001.Ch09.pdf> [retrieved on 20140610]
- LAI ET AL: "Red TL of quartz extracted from Chinese loess: Bleachability and saturation dose", RADIATION MEASUREMENTS, ELSEVIER, AMSTERDAM, NL, vol. 41, no. 7-8, 1 August 2006 (2006-08-01), pages 836 - 840, XP005711440, ISSN: 1350-4487, DOI: 10.1016/J.RADMEAS.2006.04.017

## Beschreibung

Die Erfindung betrifft ein Verfahren zur Trennung eines Gasgemisches, das zumindest Methan und Kohlenstoffdioxid enthält. Bei dem Gasgemisch handelt es sich insbesondere um ein durch Fermentation aus Biomasse erzeugtes Gasgemisch. Derartige Gasgemische werden auch als Biogas bezeichnet. Darüber hinaus betrifft die Erfindung eine Vorrichtung zur Trennung eines solchen Gasgemisches.

Verfahren und Vorrichtungen der eingangs genannten Art sind aus der Praxis und aus dem Stand der Technik in unterschiedlichen Ausführungsformen grundsätzlich bekannt. WO 2013/087046 A1 beschreibt ein Verfahren und eine Anlage zur Abtrennung von Kohlenstoffdioxid aus methanhaltigen Rohgasen. DE 10 2008 060 310 A1 betrifft ein Verfahren und eine Anlage zur Reinigung von Rohgasen, insbesondere von Biogas, zur Gewinnung von Methan. DE 10 2005 051 952 B3 offenbart ein Verfahren zur Herstellung von Methan und flüssigem Kohlenstoffdioxid aus Raffinerie- und/oder Biogas. DE 10 2008 025 971 A1 betrifft ein Verfahren und eine Anlage zur Reinigung von Biogas zur Gewinnung von Methan. Die Publikation LAURA ANNAMARIA PELLEGRINI ET AL: "Biogas to liquefied biomethane via cryogenic upgrading technologies", RENEWABLE ENERGY, Bd. 124, 31. August 2018 (2018-08-31), Seiten 75-83, XP055711440, GB; ISSN: 0960-1481, DOI: 10.1016/j.renene.2017.08.007 beschreibt die Erzeugung von Biomethan aus Biogas. Zur Erzeugung von Biogas wird in Biogasanlagen üblicherweise Biomasse unter geeigneten Fermentationsbedingungen fermentiert. Dabei werden Gasgemische erhalten, die zumindest Methan und Kohlenstoffdioxid aufweisen und darüber hinaus - in Abhängigkeit von den Fermentationsbedingungen - Wasserstoff, sowie Nebenkomponenten wie Schwefelverbindungen enthalten können. Die Gasgemische können direkt zur Strom- und Wärmeerzeugung eingesetzt werden. Alternativ können die Gasgemische zur Gewinnung von biogenem Methan verwendet werden. Um aus dem Biogas bzw. dem Gasgemisch Methan in einer den Anforderungen entsprechenden Reinheit gewinnen zu können, sind allerdings Trennungsschritte erforderlich.

Bei den aus der Praxis bekannten Verfahren und Vorrichtungen hat sich gezeigt, dass die entsprechenden Trennungsschritte sehr aufwendig und insbesondere kostenaufwendig sind. Eine Trennung der durch Fermentation aus Biomasse erzeugten Gasgemische zur Gewinnung von biogenem Methan mit den aus der Praxis bekannten Maßnahmen, beispielsweise mittels Druckwechseladsorption oder mittels Aminwäsche, ist daher nicht immer wirtschaftlich. Zudem hat sich gezeigt, dass die Funktionssicherheit der Gastrennung bei den bekannten Verfahren teilweise zu wünschen übrig lässt bzw. dass eine zufriedenstellende Reinheit der Produktgase nur mit sehr hohem Trennaufwand möglich ist. Insbesondere für kleinere Biogasanlagen ist eine Gastrennung mit den bekannten Maßnahmen oftmals unwirtschaftlich. - Insoweit besteht Verbesserungsbedarf.

Der Erfindung liegt demgegenüber das technische Problem zugrunde, ein Verfahren der eingangs genannten Art anzugeben, mit dem die vorstehend beschriebenen Nachteile vermieden werden können und mit dem insbesondere eine Trennung eines - vorzugsweise durch Fermentation aus Biomasse erzeugten - Gasgemisches, das zumindest Methan und Kohlenstoffdioxid enthält, möglich ist, die einfach, funktionssicher und wenig aufwendig bzw. kostenaufwendig durchgeführt werden kann. Darüber hinaus liegt der Erfindung das technische Problem zugrunde, eine Vorrichtung zur Trennung eines vorzugsweise durch Fermentation aus Biomasse erzeugten Gasgemisches anzugeben.

Zur Lösung des technischen Problems lehrt die Erfindung ein Verfahren zur Trennung eines, insbesondere durch Fermentation aus Biomasse erzeugten, Gasgemisches, das zumindest Methan und Kohlenstoffdioxid enthält, wobei das Gasgemisch in einem Druckbehälter, in dem ein Druck im Bereich von 2 bar bis 30 bar, vorzugsweise von 5 bar bis 25 bar, bevorzugt von 10 bar bis 20 bar herrscht, mit einer flüssigen Phase in Kontakt gebracht wird, sodass sich zumindest ein Teil des Kohlenstoffdioxids in der flüssigen Phase löst, wobei das gelöste Kohlenstoffdioxid in einer Strippingeinrichtung durch zumindest ein Strippingmittel als Teil zumindest eine Stripping-Stoffstromes zumindest teilweise aus der flüssigen Phase abgetrennt wird, insbesondere kontinuierlich, und wobei das Strippingmittel ein Alkan mit zwei bis fünf Kohlenstoffatomen ist.

Es ist bevorzugt, dass sich das in dem Gasgemisch enthaltene Kohlenstoffdioxid vollständig bzw. im Wesentlichen vollständig in der flüssigen Phase löst. Es ist weiterhin bevorzugt, dass das gelöste Kohlenstoffdioxid in der Strippingeinrichtung durch das zumindest eine Strippingmittel als Teil zumindest eines Stripping-Stoffstromes vollständig bzw. im Wesentlichen vollständig aus der flüssigen Phase abgetrennt wird.

Es liegt im Rahmen der Erfindung, dass das Gasgemisch neben Methan und Kohlenstoffdioxid Nebenkomponenten enthält. Bei den Nebenkomponenten kann es sich beispielsweise um Ammoniak und/oder um Schwefelverbindungen, insbesondere um Schwefelwasserstoff handeln. Zweckmäßigerweise lösen sich auch die in dem Gasgemisch enthaltenen Nebenkomponenten zumindest teilweise, vorzugsweise vollständig bzw. im Wesentlichen vollständig in der flüssigen Phase und werden bevorzugt mit dem Strippingmittel als Teil des Stripping-Stoffstromes zumindest teilweise, vorzugsweise vollständig bzw. im Wesentlichen vollständig, aus der flüssigen Phase abgetrennt, insbesondere kontinuierlich abgetrennt. Gemäß einer bevorzugten Ausführungsform der Erfindung weist das (ungetrennte) Gasgemisch zwischen 1 Vol.-% und 50 Vol.-%, vorzugsweise zwischen 10 Vol.-% und 45 Vol.-%, bevorzugt zwischen 15 Vol.-% und 40 Vol.-% Kohlenstoffdioxid auf. Weiter bevorzugt weist das (ungetrennte) Gasgemisch zwischen 45 Vol.-% und 95 Vol.-%, vorzugsweise zwischen 50 Vol.-% und 85 Vol.-%, bevorzugt zwischen 55 Vol.-% und 80 Vol.-% Methan auf.

Mit dem Begriff Druck ist im Rahmen der Erfindung insbesondere der Relativdruck bzw. Überdruck gemeint. Es ist bevorzugt, dass während des gesamten erfindungsgemäßen Verfahrens bzw. in der gesamten erfindungsgemäßen Vorrichtung ein Druck im Bereich von 2 bar bis 30 bar, vorzugsweise von 5 bar bis 25 bar, bevorzugt von 10 bar bis 20 bar bei herrscht. Empfohlenermaßen herrscht zumindest auch in der Strippingeinrichtung ein Druck in diesem Bereich. Ganz besonders bevorzugt herrscht während des gesamten erfindungsgemäßen Verfahrens bzw. in der gesamten erfindungsgemäßen Vorrichtung einen Druck, der dem Druck in dem Druckbehälter entspricht bzw. der im Wesentlichen dem Druck in dem Druckbehälter entspricht.

Die flüssige Phase, mit der das Gasgemisch im Rahmen des erfindungsgemäßen Verfahrens in Kontakt gebracht wird, ist insbesondere eine wässrige flüssige Phase. Es ist möglich, dass die flüssige Phase aus Wasser besteht bzw. im Wesentlichen besteht. Bei der Strippingeinrichtung handelt es sich zweckmäßigerweise um eine Strippingkolonne. Stripping meint im Rahmen der Erfindung insbesondere ein Trennverfahren zur Abtrennung zumindest eines Stoffes aus einer flüssigen Phase mit einem insbesondere gasförmigen Strippingmittel, bei dem der zumindest eine abzutrennende Stoff aus der flüssigen Phase in die Gasphase überführt wird und die Strippingeinrichtung besonders bevorzugt zusammen mit dem Strippingmittel als Stripping-Stoffstrom verlässt.

Eine besonders bevorzugte Ausführungsform des erfindungsgemäßen Verfahrens ist dadurch gekennzeichnet, dass der Druckbehälter ein Druck-Fermenter ist, in dem vorzugsweise das Gasgemisch durch Fermentation aus Biomasse erzeugt wird und wobei die flüssige Phase zweckmäßigerweise eine flüssige Fermenterphase ist. In dem Druck-Fermenter kann die Fermentation im Rahmen des erfindungsgemäßen Verfahrens im Batch-Betrieb oder im kontinuierlichen Betrieb durchgeführt werden. Die flüssige Fermenterphase ist zweckmäßigerweise eine wässrige flüssige Fermenterphase. Es ist möglich, dass die flüssige Fermenterphase aus Wasser besteht bzw. im Wesentlichen besteht, wobei in der flüssigen Fermenterphase Biomasse-Partikel suspendiert sein können.

Die Erfindung hat erkannt, dass sich durch den Kontakt des Gasgemisches mit einer flüssigen Phase in einem Druckbehälter zumindest ein Teil des Kohlenstoffdioxids und vorzugsweise zumindest einen Teil von gegebenenfalls in dem Gasgemisch enthaltenen Nebenkomponenten in der flüssigen Phase löst. Die in der flüssigen Phase gelösten Gase werden dann in der Strippingeinrichtung als Teil zumindest eines Stripping-Stoffstromes zumindest teilweise aus der flüssigen Phase abgetrennt. Zweckmäßigerweise wird die flüssige Phase anschließend in den Druckbehälter zurückgeführt. Somit wird vorzugsweise von dem Druckbehälter zu der Strippingeinrichtung eine Kohlenstoffdioxid-reiche flüssige Phase geführt und bevorzugt wird von der Strippingeinrichtung zu dem Druckbehälter eine Kohlenstoffdioxid-arme flüssige Phase zurückgeführt. In dem Druckbehälter verbleibt vorzugsweise das in dem Gasgemisch enthaltene Methan, das zweckmäßigerweise abgeführt und als biogenes Methan verwertet werden kann.

Eine Ausführungsform des erfindungsgemäßen Verfahrens ist dadurch gekennzeichnet, dass die Biomasse in einem Vorfermenter zunächst vorfermentiert wird, wobei insbesondere Wasserstoff erzeugt wird. Die Vorfermentierung ist zweckmäßigerweise der Gastrennung in dem Druckbehälter bzw. der Erzeugung und Trennung des Gasgemisches in dem Druck-Fermenter vorgeschaltet. Die vorfermentierte Biomasse wird anschließend bevorzugt zur weiteren Fermentation in den Druck-Fermenter überführt. Der Wasserstoff verbleibt empfohlenermaßen in dem Vorfermenter und fällt somit als zusätzliches wertvolles Produkt im Rahmen dieser Ausführungsform des erfindungsgemäßen Verfahrens an.

Eine besonders bevorzugte Ausführungsform der Erfindung ist dadurch gekennzeichnet, dass die flüssige Phase in einem Kreislauf zwischen dem Druckbehälter und der Strippingeinrichtung geführt wird, insbesondere kontinuierlich geführt wird. Dieser Ausführungsform liegt die Erkenntnis zugrunde, dass in der flüssigen Phase gelöstes Kohlenstoffdioxid und gegebenenfalls in der flüssigen Phase gelöste Nebenkomponenten in der Strippingeinrichtung zumindest teilweise aus der flüssigen Phase abgetrennt werden und dass die flüssige Phase anschließend in den Druckbehälter zurückgeführt werden kann. Wenn der Druckbehälter ein Druck-Fermenter ist, wird somit die flüssige Fermenterphase in einem Kreislauf zwischen dem Druck-Fermenter und der Strippingeinrichtung geführt, insbesondere kontinuierlich geführt. Dann wird in dem Druck-Fermenter durch die fortlaufende Fermentation kontinuierlich ein Gasgemisch erzeugt, das zumindest Kohlenstoffdioxid und Methan enthält. Das Kohlenstoffdioxid löst sich in der flüssigen Fermenterphase und wird kontinuierlich durch die Kreislaufführung zwischen dem Druck-Fermenter und der Strippingeinrichtung mittels Stripping aus der flüssigen Fermenterphase abgetrennt. Auf diese Weise wird in dem Druck-Fermenter kontinuierlich Methan bereitgestellt.

Es ist bevorzugt, dass das Strippingmittel in der Strippingeinrichtung mit der flüssigen Phase in Kontakt gebracht wird und vorzugsweise im Gegenstrom zu der flüssigen Phase geführt wird. Gegenstrom meint in diesem Zusammenhang insbesondere, dass die flüssige Phase und das Strippingmittel die Strippingeinrichtung zumindest abschnittsweise in entgegengesetzter Richtung durchströmen. Auf diese Weise kann die Überführung des in der flüssigen Phase gelösten Kohlenstoffdioxids in die Gasphase bzw. in den Stripping-Stoffstrom besonders funktionssicher durchgeführt werden. Dazu wird das Strippingmittel der Strippingeinrichtung zweckmäßigerweise in einem unteren Abschnitt zugeführt und die flüssige Phase wird der Strippingeinrichtung empfohlenermaßen oberhalb des Zugabepunktes für das Strippingmittel zugeführt. Zweckmäßigerweise wird die flüssige Phase der Strippingeinrichtung anschließend in einem unteren Abschnitt bzw. am unteren Ende entnommen und insbesondere zurück zu dem Druckbehälter geführt. Es ist grundsätzlich im Rahmen der Erfindung möglich, dass das Strippingmittel und die flüssige Phase in der Strippingeinrichtung im Gleichstrom geführt werden.

Gemäß einer sehr bevorzugten Ausführungsform der Erfindung ist das Strippingmittel zumindest bei der Einführung in die Strippingeinrichtung gasförmig. Zweckmäßigerweise ist der aus der Strippingeinrichtung austretende Stripping-Stoffstrom gasförmig. Der Stripping-Stoffstrom besteht vorzugsweise aus dem Strippingmittel und den durch das Stripping aus der flüssigen Phase abgetrennten Gasen wie Kohlenstoffdioxid und gegebenenfalls Nebenkomponenten. Es wurde bereits vorstehend erläutert, dass das Strippingmittel der Strippingeinrichtung zweckmäßigerweise in einem unteren Abschnitt zugegeben wird. Es empfiehlt sich in diesem Zusammenhang, dass das Strippingmittel bzw. das gasförmige Strippingmittel mittels zumindest einer Düse in die Strippingeinrichtung, insbesondere in einen unteren Abschnitt der Strippingeinrichtung, eingedüst wird. Der gasförmige Zustand des Strippingmittels zumindest bei der Einführung in die Strippingeinrichtung wird zweckmäßigerweise durch die in der Vorrichtung bzw. in der Strippingeinrichtung herrschenden Druckverhältnisse und/oder durch die Temperatur des Strippingmittels realisiert.

Erfindungsgemäß ist das Strippingmittel ein Alkan mit zwei bis fünf Kohlenstoffatomen, besonders bevorzugt zumindest ein aus der Gruppe: "Ethan, Propan, n-Butan, iso-Butan" ausgewähltes Strippingmittel und ganz besonders bevorzugt iso-Butan. Die Verwendung von iso-Butan als Strippingmittel hat sich im Rahmen der Erfindung besonders bewährt. Dem Einsatz eines Kohlenwasserstoffes bzw. eines Alkans mit zwei bis fünf Kohlenstoffatomen als Strippingmittel und insbesondere der Verwendung von iso-Butan liegt zunächst die Erkenntnis zugrunde, dass diese Strippingmittel mit verhältnismäßig geringem Aufwand als gasförmiges Strippingmittel in der Strippingeinrichtung gehandhabt werden können. Gleichzeitig ist eine Verflüssigung dieser Strippingmittel, insbesondere des iso-Butans für die weitere Aufarbeitung des Stripping-Stoffstromes mit verhältnismäßig geringem Aufwand möglich. Darüber hinaus hat sich gezeigt, dass diese Strippingmittel und insbesondere iso-Butan eine sehr zuverlässige Abtrennung des Kohlenstoffdioxids und gegebenenfalls der Nebenkomponenten aus der flüssigen Phase ermöglichen. Zudem sind die bevorzugten Strippingmittel und insbesondere iso-Butan auch aus umwelttechnischen Gesichtspunkten vorteilhaft.

Es wurde bereits oben stehend erläutert, dass der aus der Strippingeinrichtung austretende Stripping-Stoffstrom zumindest das Strippingmittel und zumindest Kohlenstoffdioxid, sowie gegebenenfalls Nebenkomponenten aufweist. Eine ganz besonders bevorzugte Ausführungsform der Erfindung ist dadurch gekennzeichnet, dass der aus der Strippingeinrichtung austretende Stripping-Stoffstrom einer Trenneinrichtung, vorzugsweise einer Rektifikationskolonne, zugeführt wird. Zweckmäßigerweise wird in der Trenneinrichtung ein Kohlenstoffdioxid-haltiger Stoffstrom von dem Stripping-Stoffstrom abgetrennt, insbesondere kontinuierlich abgetrennt. Es empfiehlt sich, dass der Stripping-Stoffstrom der Trenneinrichtung unterhalb eines oberen Endes der Trenneinrichtung zugeführt wird. Gemäß einer Ausführungsform wird der Stripping-Stoffstrom der Trenneinrichtung in Bezug auf die Länge der Trenneinrichtung in einem Abstand von dem oberen Ende der Trenneinrichtung zugeführt, der zwischen 40 % und 60 %, bevorzugt zwischen 45 % und 55 % der Länge der Trenneinrichtung entspricht. Gemäß einer alternativen Ausführungsform wird der Stripping-Stoffstrom der Trenneinrichtung an einem unteren Ende der Trenneinrichtung zugeführt.

In der Trenneinrichtung bzw. in der Rektifikationskolonne erfolgt in Richtung des oberen Endes der Trenneinrichtung zweckmäßigerweise eine Anreicherung von Kohlenstoffdioxid. Empfohlenermaßen fällt am oberen Ende der Trenneinrichtung reines bzw. im Wesentlichen reines Kohlenstoffdioxid an. Es ist außerdem bevorzugt, dass die Trenneinrichtung zumindest im Bereich ihres oberen Endes gekühlt wird und/oder dass die Trenneinrichtung zumindest im Bereich ihres unteren Endes beheizt wird. Auf diese Weise kann der Trennvorgang in der Trenneinrichtung kontrolliert werden. Die Begriffe oberes Ende und unteres Ende der Trenneinrichtung beziehen sich im Rahmen der Erfindung insbesondere auf eine Trenneinrichtung, die in ihrem Betriebszustand vertikal bzw. im Wesentlichen vertikal ausgerichtet ist, beispielsweise auf eine Rektifikationskolonne. Wenn es sich bei der Trenneinrichtung um eine im Betriebszustand horizontal bzw. im Wesentlichen horizontal ausgerichtete Trenneinrichtung handelt, ist mit dem oberen Ende insbesondere das Ende, an dem nach dem Trennvorgang das Kohlenstoffdioxid anfällt und mit dem unteren Ende insbesondere das dem oberen Ende gegenüberliegende Ende gemeint. Mit dem Begriff Länge der Trenneinrichtung ist im Rahmen der Erfindung insbesondere die größte Längserstreckung zwischen dem unteren Ende und dem oberen Ende der Trenneinrichtung gemeint.

Es empfiehlt sich, dass der Kohlenstoffdioxid-haltige Stoffstrom der Trenneinrichtung an einem oberen Ende entnommen wird und vorzugsweise anschließend einer Speichereinrichtung zugeführt wird. Bei der Speichereinrichtung handelt es sich vorzugsweise um eine Druckgasflasche. Wenn die Trenneinrichtung gemäß bevorzugter Ausführungsform eine Rektifikationskolonne ist, handelt es sich bei dem oberen Ende der Trenneinrichtung vorzugsweise um den Kopf der Rektifikationskolonne. Es ist bevorzugt, dass der Kohlenstoffdioxid-haltige Stoffstrom Kohlenstoffdioxid als Hauptkomponente aufweist und insbesondere zu mindestens 95 Vol.-%, bevorzugt zu mindestens 97 Vol.-%, besonders bevorzugt zu mindestens 98 Vol.-% aus Kohlenstoffdioxid besteht. Gemäß einer Ausführungsform besteht der Kohlenstoffdioxid-haltige Stoffstrom vollständig bzw. im Wesentlichen vollständig aus Kohlenstoffdioxid. Es ist bevorzugt, dass der Kohlenstoffdioxid-haltige Stoffstrom bzw. das darin enthaltene Kohlenstoffdioxid nach der Entnahme aus der Trenneinrichtung verflüssigt wird, vorzugsweise mittels eines Kühlmittels verflüssigt wird. Dieser Ausführungsform liegt die Erkenntnis zugrunde, dass das in dem Kohlenstoffdioxid-haltigen Stoffstrom enthaltene Kohlenstoffdioxid durch die Verflüssigung weiter isoliert werden kann. Insbesondere können Reste von Methan, die sich in dem Druckbehälter in der flüssigen Phase lösen und mit dem Strippingmittel in die Trenneinrichtung eingeführt werden in diesem Schritt abgetrennt und vorzugsweise in den Druckbehälter zurückgeführt werden. Darüber hinaus kann das verflüssigte Kohlenstoffdioxid besonders einfach und funktionssicher einer Speichereinrichtung, beispielsweise einer Druckgasflasche zugeführt werden. Mit dem erfindungsgemäßen Trennverfahren lässt sich somit technisch reines Kohlenstoffdioxid aus dem Gasgemisch, das zumindest Methan und Kohlenstoffdioxid enthält, abtrennen. Es liegt im Übrigen im Rahmen der Erfindung, dass ein Teil des verflüssigten Kohlenstoffdioxids zur Kühlung der Trenneinrichtung, vorzugsweise zumindest im Bereich des oberen Endes der Trenneinrichtung, verwendet wird.

Es liegt weiterhin im Rahmen des erfindungsgemäßen Verfahrens, dass das Strippingmittel in der Trenneinrichtung als flüssiges Strippingmittel anfällt, vorzugsweise an einem unteren Ende der Trenneinrichtung anfällt, und bevorzugt in die Strippingeinrichtung zurückgeführt wird. Das Strippingmittel wird somit in der Trenneinrichtung zweckmäßigerweise verflüssigt. Wenn es sich bei der Trenneinrichtung gemäß bevorzugter Ausführungsform der Erfindung um eine Rektifikationskolonne handelt, fällt das Strippingmittel vorzugsweise im Kolonnensumpf, insbesondere als flüssiges Strippingmittel, an und wird besonders bevorzugt in die Strippingeinrichtung zurückgeführt. Dieser Ausführungsform liegt die Erkenntnis zugrunde, dass in der Trenneinrichtung eine sehr effiziente Abtrennung eines Kohlenstoffdioxid-haltigen Stoffstromes bzw. des Kohlenstoffdioxids aus dem Stripping-Stoffstrom möglich ist und dass vorzugsweise am unteren Ende der Trenneinrichtung das Strippingmittel und gegebenenfalls Nebenkomponenten anfallen. Das flüssige bzw. verflüssigte Strippingmittel wird zweckmäßigerweise in die Strippingeinrichtung zurückgeführt, sodass zwischen der Strippingeinrichtung und der Trenneinrichtung empfohlenermaßen eine Kreislaufführung, insbesondere eine kontinuierliche Kreislaufführung, erfolgt, bei der der Stripping-Stoffstrom aus der Strippingeinrichtung austritt und in die Trenneinrichtung eingeführt wird und bei der zweckmäßigerweise das Strippingmittel aus der Trenneinrichtung austritt und in die Strippingeinrichtung zurückgeführt wird. Die erfindungsgemäßen Trennungsschritte ermöglichen somit eine Kreislaufführung der flüssigen Phase zwischen dem Druckbehälter und der Strippingeinrichtung und/oder eine Kreislaufführung des Strippingmittels zwischen der Strippingeinrichtung und der Trenneinrichtung. Mittels der erfindungsgemäßen Trennungsschritte lassen sich Methan und Kohlenstoffdioxid in einer sehr zufriedenstellenden Reinheit abtrennen.

Es ist möglich, dass in der Trenneinrichtung ein Druck im Bereich von 2 bar bis 30 bar, vorzugsweise von 5 bar bis 25 bar, bevorzugt von 10 bar bis 20 bar, herrscht. Besonders bevorzugt herrscht in der Trenneinrichtung ein Druck, der dem Druck in dem Druckbehälter und/oder in der Strippingeinrichtung entspricht bzw. im Wesentlichen entspricht, wobei dies zweckmäßigerweise eine Druckabweichung zur Realisierung des Trennvorganges bzw. der Rektifikation einschließt.

Es ist im Rahmen der Erfindung möglich, dass der Druckaufbau im Druckbehälter durch technische Kompression und/oder durch die Erzeugung des Gasgemisches aus Biomasse mittels Fermentation erfolgt.

Zur Lösung des technischen Problems lehrt die Erfindung weiterhin eine Vorrichtung zur Trennung eines, vorzugsweise durch Fermentation aus Biomasse erzeugten, Gasgemisches, zur Durchführung eines vorstehend beschriebenen Verfahrens, wobei die Vorrichtung einen Druckbehälter aufweist, in dem das Gasgemisch, das zumindest Methan und Kohlenstoffdioxid enthält, unter Druck mit einer flüssigen Phase in Kontakt gebracht werden kann, wobei die Vorrichtung eine mit dem Druckbehälter verbundene Strippingeinrichtung aufweist, mit der in der flüssigen Phase gelöstes Kohlenstoffdioxid durch zumindest ein Strippingmittel als Teil zumindest eines Stripping-Stoffstromes zumindest teilweise aus der flüssigen Phase abtrennbar ist, insbesondere kontinuierlich, und wobei das Strippingmittel (5) ein Alkan mit zwei bis fünf Kohlenstoffatomen ist.

Es liegt im Rahmen der Erfindung, dass der Druckbehälter ein Druck-Fermenter ist, in dem vorzugsweise das Gasgemisch durch Fermentation aus Biomasse erzeugbar ist und wobei die flüssige Phase vorzugsweise eine flüssige Fermenterphase ist. Es ist außerdem möglich, dass die erfindungsgemäße Vorrichtung einen dem Druckbehälter vorgeschalteten Vorfermenter aufweist, in dem die Biomasse vorfermentierbar ist.

Eine besonders bevorzugte Ausführungsform der erfindungsgemäßen Vorrichtung ist dadurch gekennzeichnet, dass der Druckbehälter über zumindest zwei Verbindungsleitungen derart mit der Strippingeinrichtung verbunden ist, dass die flüssige Phase in einem Kreislauf zwischen dem Druckbehälter und der Strippingeinrichtung führbar ist. Zweckmäßigerweise führt eine erste, für eine Kohlenstoffdioxid-reiche flüssige Phase vorgesehene Verbindungsleitung von dem Druckbehälter und insbesondere von einem unteren Abschnitt des Druckbehälters zu der Strippingeinrichtung. Mittels der ersten Verbindungsleitung wird eine Kohlenstoffdioxid-reiche flüssige Phase der Strippingeinrichtung zugeführt. Bevorzugt führt eine zweite Verbindungsleitung von der Strippingeinrichtung und insbesondere von einem unteren Abschnitt der Strippingeinrichtung zurück zu dem Druckbehälter. Mittels dieser zweiten Verbindungsleitung wird die Kohlenstoffdioxid-arme flüssige Phase aus der Strippingeinrichtung zurück in den Druckbehälter geführt. Die flüssige Phase ist mittels der zumindest zwei Verbindungsleitungen somit im Kreislauf zwischen dem Druckbehälter und der Strippingeinrichtung führbar und insbesondere kontinuierlich in einem solchen Kreislauf führbar.

Es hat sich bewährt, dass die erfindungsgemäße Vorrichtung zumindest eine der Strippingeinrichtung nachgeschaltete Trenneinrichtung aufweist, mit der ein Kohlenstoffdioxid-haltiger Stoffstrom von dem Stripping-Stoffstrom abtrennbar ist. Gemäß besonders bevorzugter Ausführungsform der erfindungsgemäßen Vorrichtung ist die Trenneinrichtung eine Rektifikationskolonne, vorzugsweise eine Füllkörperkolonne und/oder eine Glockenbodenkolonne. Eine Reklifikationskolonne weist im Rahmen der Erfindung insbesondere einen das obere Ende bildenden Kolonnenkopf und einen das untere Ende bildenden Kolonnensumpf auf. Der Kohlenstoffdioxid-haltige Stoffstrom ist der Trenneinrichtung insbesondere an dem oberen Ende entnehmbar. Das Strippingmittel fällt in der Trenneinrichtung insbesondere als flüssiges bzw. verflüssigtes Strippingmittel, bevorzugt am unteren Ende der Trenneinrichtung an.

Es liegt im Rahmen der Erfindung, dass die Strippingeinrichtung über zumindest zwei Verbindungsleitungen derart mit der Trenneinrichtung verbunden ist, dass das Strippingmittel in einem Kreislauf zwischen der Strippingeinrichtung und der Trenneinrichtung führbar ist. Zweckmäßigerweise ist das Strippingmittel kontinuierlich in einem Kreislauf zwischen der Strippingeinrichtung und der Trenneinrichtung führbar. Empfohlenermaßen wird mittels zumindest einer ersten Verbindungsleitung der das Strippingmittel aufweisende Stripping-Stoffstrom von der Strippingeinrichtung, insbesondere von einem oberen Abschnitt der Strippingeinrichtung, zu der Trenneinrichtung geführt und in die Trenneinrichtung eingeführt. Weiter bevorzugt wird mittels zumindest einer zweiten Verbindungsleitung das flüssige bzw. verflüssigte Strippingmittel von der Trenneinrichtung, insbesondere von dem unteren Ende der Trenneinrichtung, zurück in die Strippingeinrichtung, insbesondere zu einem unteren Abschnitt der Strippingeinrichtung, geführt und in die Strippingeinrichtung zurückgeführt. Dabei wird das Strippingmittel zweckmäßigerweise aufgeheizt, sodass es als gasförmiges Strippingmittel in die Strippingeinrichtung einführbar ist. Dieser Ausführungsform liegt die Erkenntnis zugrunde, dass das Strippingmittel zwischen der Strippingeinrichtung und der Trenneinrichtung im Kreislauf geführt werden kann, insbesondere kontinuierlich im Kreislauf geführt werden kann und somit wiederverwendet werden kann.

Es ist weiterhin bevorzugt, dass die Vorrichtung eine Kälteeinrichtung zur Verflüssigung des abgetrennten Kohlenstoffdioxid-haltigen Stoffstromes bzw. des darin enthaltenen Kohlenstoffdioxids aufweist. Die Kälteeinrichtung ist vorzugsweise einem oberen Ende der Trenneinrichtung zugeordnet. Wenn es sich bei der Trenneinrichtung gemäß bevorzugter Ausführungsform um eine Rektifikationskolonne handelt, ist die Kälteeinrichtung zweckmäßigerweise dem Kolonnenkopf zugeordnet. Mit der Zuordnung der Kälteeinrichtung zu dem oberen Ende der Trenneinrichtung ist im Rahmen der Erfindung insbesondere gemeint, dass der aus der Trenneinrichtung austretende Kohlenstoffdioxid-haltige Stoffstrom bzw. das darin enthaltene Kohlenstoffdioxid mittels der Kälteeinrichtung verflüssigt werden kann. Es ist bevorzugt, dass ein Teil des verflüssigten Kohlenstoffdioxids zur Kühlung der Trenneinrichtung zurück zu dem oberen Ende der Trenneinrichtung zurückgeführt wird. Das übrige verflüssigte Kohlenstoffdioxid ist zweckmäßigerweise einer Speichereinrichtung, beispielsweise einer Druckgasflasche, zuführbar.

Der Erfindung liegt die Erkenntnis zugrunde, dass mit dem erfindungsgemäßen Verfahren bzw. mit der erfindungsgemäßen Vorrichtung eine einfache und funktionssichere Trennung von Gasgemischen, insbesondere von durch Fermentation aus Biomasse erzeugten Gasgemischen, die zumindest Methan und Kohlenstoffdioxid enthalten, realisierbar ist. In dem Druckbehälter wird aufgrund der Druckverhältnisse Kohlenstoffdioxid durch Lösung in einer flüssigen Phase aus dem Gasgemisch abgetrennt, sodass Methan in einer allen Anforderungen entsprechenden Reinheit in dem Druckbehälter verbleibt. Das in der flüssigen Phase gelöste Kohlenstoffdioxid wird erfindungsgemäß in einer Strippingeinrichtung aus der flüssigen Phase abgetrennt. In einer Trenneinrichtung wird gemäß bevorzugter Ausführungsform aus dem resultierenden Stripping-Stoffstrom ein Kohlenstoffdioxid-haltiger Stoffstrom, der Kohlenstoffdioxid als Hauptkomponente enthält, abgetrennt. Mittels des erfindungsgemäßen Verfahrens und mittels der erfindungsgemäßen Vorrichtung lassen sich somit Methan und Kohlenstoffdioxid in einer hohen Reinheit aus dem Gasgemisch abtrennen. Das erfindungsgemäße Verfahren und die erfindungsgemäße Vorrichtung zeichnen sich dabei insbesondere durch ihre wenig aufwendigen Maßnahmen aus, die nichtsdestoweniger eine überraschend funktionssichere Trennung des Gasgemisches ermöglichen, sodass eine hohe Reinheit des Methans und des Kohlenstoffdioxids gewährleistet ist. Zu betonen ist weiterhin, dass sich die erfindungsgemäßen Maßnahmen durch eine hohe Effizienz auszeichnen, sodass das Verfahren und die Vorrichtung unabhängig von dem Maßstab der Biogasanlage sehr wirtschaftlich zur Gastrennung eingesetzt werden können. Im Ergebnis kann durch die erfindungsgemäßen Maßnahmen eine funktionssichere Gastrennung erfolgen, sodass Gasprodukte mit hoher Reinheit erhalten werden, wobei das Trennverfahren aufgrund der sehr effizienten Trennungsschritte sehr wirtschaftlich ist.

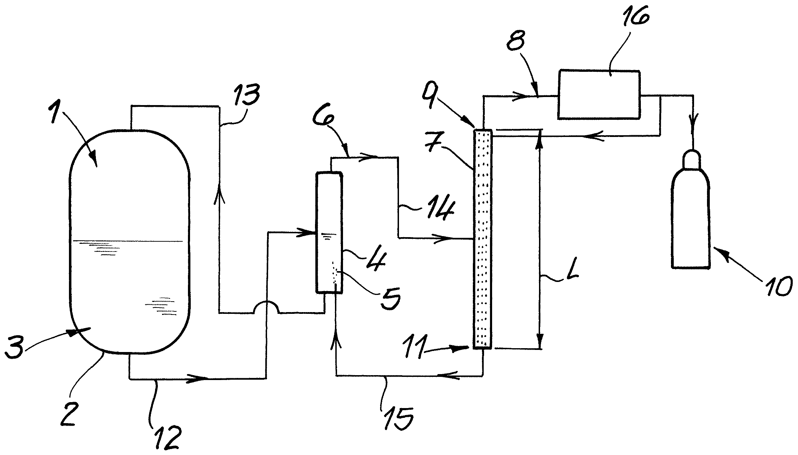

Nachfolgend wird die Erfindung anhand einer lediglich ein Ausführungsbeispiel darstellenden Zeichnung näher erläutert. Die einzige Figur zeigt in schematischer Darstellung ein Fließschema einer erfindungsgemäßen Vorrichtung zur Durchführung des erfindungsgemäßen Verfahrens.

Die Vorrichtung weist einen Druckbehälter 2 auf, der bevorzugt und im Ausführungsbeispiel als Druck-Fermenter ausgebildet ist. Im Rahmen des erfindungsgemäßen Verfahrens wird in dem als Druck-Fermenter ausgebildeten Druckbehälter 2 durch Fermentation aus Biomasse ein Gasgemisch 1 erzeugt, das zumindest Methan und Kohlenstoffdioxid enthält. Das Gasgemisch mag zusätzlich Nebenkomponenten wie Schwefelwasserstoff enthalten. In dem Druckbehälter 2 bzw. in dem Druck-Fermenter herrscht erfindungsgemäß ein Druck im Bereich von 2 bar bis 30 bar, vorzugsweise von 10 bar bis 20 bar. Gemäß einer empfohlenen Ausführungsform der Erfindung und im Ausführungsbeispiel erfolgt der Druckaufbau in dem Druckbehälter 2 durch die Erzeugung des Gasgemisches 1 aus Biomasse mittels Fermentation.

Das Gasgemisch 1 wird in dem Druckbehälter 2 erfindungsgemäß mit einer flüssigen Phase 3 in Kontakt gebracht, sodass sich unter den herrschenden Druckbedingungen zumindest ein Teil des Kohlenstoffdioxids in der flüssigen Phase 3 löst. Die flüssige Phase 3 ist zweckmäßigerweise und im Ausführungsbeispiel eine wässrige flüssige Phase und insbesondere eine wässrige flüssige Fermenterphase. Bevorzugt löst sich das in dem Gasgemisch 1 enthaltene Kohlenstoffdioxid vollständig bzw. im Wesentlichen vollständig in der flüssigen Phase 3. Im Übrigen lösen sich vorzugsweise auch gegebenenfalls in dem Gasgemisch 1 enthaltene Nebenkomponenten wie Schwefelwasserstoff zumindest teilweise, vorzugsweise vollständig bzw. im Wesentlichen vollständig in der flüssigen Phase 3.

In einer empfohlenermaßen und im Ausführungsbeispiel als Strippingkolonne ausgebildeten Strippingeinrichtung 4 wird das gelöste Kohlenstoffdioxid durch zumindest ein Strippingmittel 5 als Teil zumindest eines Stripping-Stoffstromes 6 zumindest teilweise, vorzugsweise vollständig bzw. im Wesentlichen vollständig aus der flüssigen Phase 3 abgetrennt, bevorzugt und im Ausführungsbeispiel kontinuierlich abgetrennt. Auch die gegebenenfalls in dem Gasgemisch 1 enthaltenen Nebenkomponenten werden zweckmäßigerweise und im Ausführungsbeispiel mit dem Strippingmittel 5 als Teil des Stripping-Stoffstromes 6 zumindest teilweise, vorzugsweise vollständig bzw. im Wesentlichen vollständig aus der flüssigen Phase 3 abgetrennt. Weiter bevorzugt und im Ausführungsbeispiel gemäß der einzigen Figur herrscht im Übrigen auch in der Strippingeinrichtung 4 ein Druck im Bereich von 2 bar bis 30 bar, bevorzugt von 10 bar bis 20 bar. Zweckmäßigerweise herrscht während des gesamten erfindungsgemäßen Verfahrens bzw. in der gesamten erfindungsgemäßen Vorrichtung ein Druck in diesem Bereich und besonders bevorzugt ein Druck, der dem Druck in dem Druckbehälter 2 entspricht bzw. der im Wesentlichen dem Druck in dem Druckbehälter 2 entspricht.

Gemäß besonders bevorzugter Ausführungsform und im Ausführungsbeispiel wird die flüssige Phase 3 kontinuierlich in einem Kreislauf zwischen dem Druckbehälter 2 und der Strippingeinrichtung 4 geführt. Die flüssige Phase 3 zirkuliert somit zweckmäßigerweise zwischen dem Druckbehälter 2 und der Strippingeinrichtung 4. Dazu weist die Vorrichtung empfohlenermaßen und im Ausführungsbeispiel zwei Verbindungsleitungen 12, 13 auf, mittels derer der Druckbehälter 2 mit der Strippingeinrichtung 4 verbunden ist. Die erste Verbindungsleitung 12 ist zweckmäßigerweise und im Ausführungsbeispiel für die Führung einer Kohlenstoffdioxid-reichen flüssigen Phase 3 von dem Druckbehälter 2 zu der Strippingeinrichtung 4 vorgesehen. Die zweite Verbindungsleitung 13 ist bevorzugt und im Ausführungsbeispiel für die Führung einer Kohlenstoffdioxid-armen flüssigen Phase 3 aus der Strippingeinrichtung 4 zurück in den Druckbehälter 2 vorgesehen. In dem als Druck-Fermenter ausgebildeten Druckbehälter 2 wird bevorzugt und im Ausführungsbeispiel durch fortlaufende Fermentation kontinuierlich ein Gasgemisch 1 erzeugt, das zumindest Kohlenstoffdioxid und Methan enthält.

Das Kohlenstoffdioxid löst sich in der flüssigen Phase 3 bzw. in der flüssigen Fermenterphase und wird kontinuierlich durch die Kreislaufführung zwischen dem Druckbehälter 2 und der Strippingeinrichtung 4 mittels Stripping aus der flüssigen Phase 3 abgetrennt. Auf diese Weise wird in dem Druckbehälter 2 kontinuierlich Methan bereitgestellt.

In der als Strippingkolonne ausgebildeten Strippingeinrichtung 4 wird das Strippingmittel 5 mit der flüssigen Phase 3 bzw. mit der flüssigen Fermenterphase in Kontakt gebracht und vorzugsweise und im Ausführungsbeispiel im Gegenstrom zu der flüssigen Phase 3 geführt. Gegenstrom meint dabei, dass die flüssige Phase 3 und das Strippingmittel 5 die Strippingeinrichtung 4 in entgegengesetzter Richtung durchströmen. Das Strippingmittel 5 wird der Strippingeinrichtung 4 zweckmäßigerweise und im Ausführungsbeispiel in einem unteren Abschnitt zugeführt. Die flüssige Phase 3 wird der Strippingeinrichtung 4 vorzugsweise oberhalb des Zugabepunktes für das Strippingmittel 5 zugeführt. Zweckmäßigerweise und im Ausführungsbeispiel wird die flüssige Phase 3 anschließend in einem unteren Abschnitt der Strippingeinrichtung 4 entnommen und mittels der zweiten Verbindungsleitung 13 zurück zu dem Druckbehälter 2 geführt.

Das Strippingmittel 5 ist gemäß bevorzugter Ausführungsform und im Ausführungsbeispiel zumindest bei der Einführung in die Strippingeinrichtung 4 gasförmig. Zweckmäßigerweise ist auch der aus der Strippingeinrichtung 4 austretende Stripping-Stoffstrom 6 gasförmig. Bevorzugt und im Ausführungsbeispiel wird das zumindest bei der Einführung in die Strippingeinrichtung 4 gasförmige Strippingmittel 5 mittels zumindest einer Düse in die Strippingeinrichtung 4 eingedüst und insbesondere in einem unteren Abschnitt der Strippingeinrichtung 4 eingedüst.

Der gasförmige Zustand des Strippingmittels 5 zumindest bei der Einführung in die Strippingeinrichtung 4 wird zweckmäßigerweise durch die in der Vorrichtung bzw. in der Strippingeinrichtung 4 herrschenden Druckverhältnisse und/oder durch die Temperatur des Strippingmittels 5 realisiert. Es ist erfindungsgemäß, dass das Strippingmittel ein Alkan mit zwei bis fünf Kohlenstoffatomen ist. Im Ausführungsbeispiel mag das Strippingmittel 5 iso-Butan sein.

Der Stripping-Stoffstrom 6 besteht vorzugsweise und im Ausführungsbeispiel zumindest aus dem Strippingmittel 5 und dem durch das Stripping aus der flüssigen Phase 3 abgetrennten Kohlenstoffdioxid, sowie gegebenenfalls aus Nebenkomponenten wie Schwefelwasserstoff. Gemäß einer ganz besonders bevorzugten Ausführungsform der Erfindung und im Ausführungsbeispiel gemäß der einzigen Figur wird der aus der Strippingeinrichtung 4 austretende Stripping-Stoffstrom 6 einer Trenneinrichtung 7, die bevorzugt und im Ausführungsbeispiel als Rektifikationskolonne ausgebildet ist, zugeführt.

In der Trenneinrichtung 7 wird bevorzugt und im Ausführungsbeispiel ein Kohlenstoffdioxid-haltiger Stoffstrom 8 von dem Stripping-Stoffstrom 6 abgetrennt, vorzugsweise und im Ausführungsbeispiel kontinuierlich abgetrennt. Dazu erfolgt in der Trenneinrichtung 7 auf dem Weg zwischen einem unteren Ende 11 und einem oberen Ende 9 der Trenneinrichtung 7 zweckmäßigerweise eine Anreicherung von Kohlenstoffdioxid. Empfohlenermaßen fällt am oberen Ende 9 der Trenneinrichtung 7 bzw. am Kolonnenkopf der Rektifikationskolonne reines bzw. im Wesentlichen reines Kohlenstoffdioxid an. Das in dem Stripping-Stoffstrom 6 enthaltene Strippingmittel 5 fällt im Rahmen der Erfindung und im Ausführungsbeispiel in der Trenneinrichtung 7 als flüssiges bzw. verflüssigtes Strippingmittel 5 an. Bevorzugt und im Ausführungsbeispiel gemäß der Figur fällt das Strippingmittel 5 in der Trenneinrichtung 7 an dem unteren Ende 11 der Trenneinrichtung 7 bzw. im Sumpf der Rektifikationskolonne als flüssiges bzw. verflüssigtes Strippingmittel 5 an. Bevorzugt und im Ausführungsbeispiel wird das flüssige bzw. verflüssigte Strippingmittel 5 anschließend zu der Strippingeinrichtung 4 zurückgeführt. Am unteren Ende der Trenneinrichtung 11 fallen im Rahmen der Erfindung im Übrigen auch gegebenenfalls in dem Stripping-Stoffstrom 6 enthaltene Nebenkomponenten wie Schwefelwasserstoff an.

Es ist bevorzugt, dass die Trenneinrichtung 7 zumindest im Bereich ihres oberen Endes 9 gekühlt wird und/oder dass die Trenneinrichtung 7 zumindest im Bereich ihres unteren Endes 11 beheizt wird. Auf diese Weise kann der Trennvorgang in der Trenneinrichtung 7 kontrolliert werden. Es ist weiterhin bevorzugt, dass in der Trenneinrichtung 7 ein Druck im Bereich von 2 bar bis 30 bar, vorzugsweise von 10 bar bis 20 bar herrscht und dass in der Trenneinrichtung 7 insbesondere ein Druck herrscht, der dem Druck in dem Druckbehälter 2 und/oder in der Strippingeinrichtung 4 entspricht bzw. im Wesentlichen entspricht, wobei dies eine geringfügige Druckabweichung zur Realisierung des Trennvorganges bzw. der Rektifikation in der Trenneinrichtung 7 bzw. in der Rektifikationskolonne einschließt.

Gemäß empfohlener Ausführungsform und im Ausführungsbeispiel wird der Stripping-Stoffstrom 6 der Trenneinrichtung 7 unterhalb des oberen Endes 9 der Trenneinrichtung 7 zugeführt. Besonders bevorzugt wird der Stripping-Stoffstrom 6 der Trenneinrichtung 7 in Bezug auf die Länge L der Trenneinrichtung 7 in einem Abstand von dem oberen Ende 9 der Trenneinrichtung 7 zugeführt, der zwischen 40 % und 60 %, besonders bevorzugt und im Ausführungsbeispiel zwischen 45 % und 55 % der Länge L der Trenneinrichtung 7 entspricht. Mit dem Begriff Länge L der Trenneinrichtung 7 ist im Rahmen der Erfindung insbesondere die größte Längserstreckung der Trenneinrichtung 7 zwischen dem unteren Ende 11 und dem oberen Ende 9 gemeint.

Zweckmäßigerweise und im Ausführungsbeispiel ist die Strippingeinrichtung 4 über zumindest zwei Verbindungsleitungen 14, 15 mit der Trenneinrichtung 7 verbunden. Das Strippingmittel 5 wird im Rahmen des erfindungsgemäßen Verfahrens und im Ausführungsbeispiel in einem Kreislauf zwischen der Strippingeinrichtung 4 und der Trenneinrichtung 7 geführt. Dabei wird zweckmäßigerweise mittels der ersten Verbindungsleitung 14 der das Strippingmittel 5 aufweisende Stripping-Stoffstrom 6 von der Strippingeinrichtung 4, insbesondere von einem oberen Abschnitt der Strippingeinrichtung 4 zu der Trenneinrichtung 7 geführt. Weiter bevorzugt und im Ausführungsbeispiel wird mittels der zweiten Verbindungsleitung 15 das flüssige bzw. verflüssigte Strippingmittel 5 von der Trenneinrichtung 7, insbesondere von dem unteren Ende 11 der Trenneinrichtung 7, zurück in die Strippingeinrichtung 4 und insbesondere zu einem unteren Abschnitt der Strippingeinrichtung 4, geführt und in die Strippingeinrichtung 4 eingeführt bzw. eingedüst. Dabei wird das flüssige bzw. verflüssigte Strippingmittel 5 auf dem Weg von der Trenneinrichtung 7 zu der Strippingeinrichtung 4 vorzugsweise beheizt, um in den gasförmigen Zustand überzugehen.

Ganz besonders bevorzugt und im Ausführungsbeispiel wird der Kohlenstoffdioxid-haltige Stoffstrom 8 der Trenneinrichtung 7 an dem oberen Ende 9 entnommen und vorzugsweise anschließend einer Speichereinrichtung 10, bevorzugt und im Ausführungsbeispiel einer Druckgasflasche, zugeführt. Es ist im Rahmen der Erfindung bevorzugt, dass der Kohlenstoffdioxid-haltige Stoffstrom 8 Kohlenstoffdioxid als Hauptkomponente aufweist und insbesondere zu mindestens 97 Vol.-%, bevorzugt zu mindestens 98 Vol.-% aus Kohlenstoffdioxid besteht. Vorzugsweise besteht der Kohlenstoffdioxid-haltige Stoffstrom 8 vollständig bzw. im Wesentlichen vollständig aus Kohlenstoffdioxid.

Empfohlenermaßen und im Ausführungsbeispiel wird der Kohlenstoffdioxid-haltige Stoffstrom 8 bzw. das darin enthaltene Kohlenstoffdioxid nach der Entnahme aus der Trenneinrichtung 7 verflüssigt, vorzugsweise mittels eines Kühlmittels verflüssigt. Dazu weist die erfindungsgemäße Vorrichtung gemäß einer bevorzugten Ausführungsform und im Ausführungsbeispiel zumindest eine Kälteeinrichtung 16 auf, die besonders bevorzugt und im Ausführungsbeispiel gemäß der Figur dem oberen Ende 9 der Trenneinrichtung 7 zugeordnet ist. Der Kohlenstoffdioxid-haltige Stoffstrom 8 bzw. das darin enthaltene Kohlenstoffdioxid kann durch die Verflüssigung vorzugsweise weiter isoliert werden. Das verflüssigte Kohlenstoffdioxid kann außerdem besonders einfach und funktionssicher der Speichereinrichtung 10 bzw. der Druckgasflasche zugeführt werden. Mit dem erfindungsgemäßen Trennverfahren wird auf diese Weise technisch reines Kohlenstoffdioxid abgetrennt und erhalten. Im Rahmen der Erfindung im Ausführungsbeispiel wird ein Teil des verflüssigten Kohlenstoffdioxids zur Kühlung der Trenneinrichtung 7 zumindest im Bereich des oberen Endes 9 der Trenneinrichtung 7 verwendet.

Mittels des erfindungsgemäßen Verfahrens bzw. mit der erfindungsgemäßen Vorrichtung ist eine einfache und funktionssichere Trennung eines Gasgemisches 1, insbesondere eines durch Fermentation aus Biomasse erzeugten Gasgemisches 1, das zumindest Methan und Kohlenstoffdioxid enthält, möglich, sodass zumindest Methan und Kohlenstoffdioxid in einer den Anforderungen entsprechenden Reinheit als abgetrennte Produktgase erhalten werden.

## Patentansprüche

1. Verfahren zur Trennung eines, insbesondere durch Fermentation aus Biomasse erzeugten, Gasgemisches (1), das zumindest Methan und Kohlenstoffdioxid enthält, wobei das Gasgemisch (1) in einem Druckbehälter (2), in dem ein Druck im Bereich von 2 bar bis 30 bar, vorzugsweise von 5 bar bis 25 bar, bevorzugt von 10 bar bis 20 bar, herrscht, mit einer flüssigen Phase (3) in
Kontakt gebracht wird, sodass sich zumindest ein Teil des Kohlenstoffdioxids in der flüssigen Phase (3) löst, wobei das gelöste Kohlenstoffdioxid in einer Strippingeinrichtung (4) durch zumindest ein Strippingmittel (5) als Teil zumindest eines Stripping-Stoffstromes (6) zumindest teilweise aus der flüssigen Phase (3) abgetrennt wird, insbesondere kontinuierlich, und wobei das Strippingmittel (5) ein Alkan mit zwei bis fünf Kohlenstoffatomen ist.

2. Verfahren nach Anspruch 1, wobei der Druckbehälter (2) ein Druck-Fermenter ist, in dem vorzugsweise das Gasgemisch (1) durch Fermentation aus Biomasse erzeugt wird und wobei die flüssige Phase (3) zweckmäßigerweise eine flüssige Fermenterphase ist.

3. Verfahren nach einem der Ansprüche 1 oder 2, wobei die flüssige Phase (3) in einem Kreislauf zwischen dem Druckbehälter (2) und der Strippingeinrichtung (4) geführt wird, insbesondere kontinuierlich geführt wird.

4. Verfahren nach einem der Ansprüche 1 bis 3, wobei das Strippingmittel (5) in der Strippingeinrichtung (4) mit der flüssigen Phase (3) in Kontakt gebracht wird und vorzugsweise im Gegenstrom zu der flüssigen Phase (3) geführt wird.

5. Verfahren nach einem der Ansprüche 1 bis 4, wobei das Strippingmittel (5) zumindest bei der Einführung in die Strippingeinrichtung (4) gasförmig ist und wobei der aus der Strippingeinrichtung (4) austretende Stripping-Stoffstrom (6) vorzugsweise gasförmig ist.

6. Verfahren nach einem der Ansprüche 1 bis 5, wobei das Strippingmittel (5) zumindest ein aus der Gruppe: "Ethan, Propan, n-Butan, iso-Butan" ausgewähltes Strippingmittel (5) und ganz besonders bevorzugt iso-Butan ist.

7. Verfahren nach einem der Ansprüche 1 bis 6, wobei der aus der Strippingeinrichtung (4) austretende Stripping-Stoffstrom (6) einer Trenneinrichtung (7), vorzugsweise einer Rektifikationskolonne, zugeführt wird, wobei in der Trenneinrichtung (7) ein Kohlenstoffdioxid-haltiger Stoffstrom (8) von dem Stripping-Stoffstrom (6) abgetrennt wird, insbesondere kontinuierlich abgetrennt wird.

8. Verfahren nach Anspruch 7, wobei der Kohlenstoffdioxid-haltige Stoffstrom (8) der Trenneinrichtung (7) an einem oberen Ende (9) entnommen wird und vorzugsweise anschließend einer Speichereinrichtung (10) zugeführt wird.

9. Verfahren nach einem der Ansprüche 7 oder 8, wobei der Kohlenstoffdioxid-haltige Stoffstrom (8) bzw. das darin enthaltene Kohlenstoffdioxid nach der Entnahme aus der Trenneinrichtung (7) verflüssigt wird, vorzugsweise mittels eines Kühlmittels verflüssigt wird.

10. Verfahren nach einem der Ansprüche 7 bis 9, wobei das Strippingmittel (5) in der Trenneinrichtung (7) als flüssiges Strippingmittel (5) anfällt, vorzugsweise an einem unteren Ende (11) der Trenneinrichtung (7) anfällt, und bevorzugt in die Strippingeinrichtung (4) zurückgeführt wird.

11. Verfahren nach einem der Ansprüche 1 bis 10, wobei der Druckaufbau im Druckbehälter (2) durch technische Kompression und/oder durch die Erzeugung des Gasgemisches (1) aus Biomasse mittels Fermentation erfolgt.

12. Vorrichtung zur Trennung eines, vorzugsweise durch Fermentation aus Biomasse erzeugten, Gasgemisches (1), zur Durchführung eines Verfahrens nach einem der Ansprüche 1 bis 11, wobei die Vorrichtung einen Druckbehälter (2) aufweist, in dem das Gasgemisch (1), das zumindest Methan und Kohlenstoffdioxid enthält, unter Druck mit einer flüssigen Phase (3) in Kontakt gebracht werden kann, wobei die Vorrichtung eine mit dem Druckbehälter (2) verbundene Strippingeinrichtung (4) aufweist, mit der in der flüssigen Phase (3) gelöstes Kohlenstoffdioxid durch zumindest ein Strippingmittel (5) als Teil zumindest eines Stripping-Stoffstromes (6) zumindest teilweise aus der flüssigen Phase (3) abtrennbar ist, insbesondere kontinuierlich, und wobei das Strippingmittel (5) ein Alkan mit zwei bis fünf Kohlenstoffatomen ist.

13. Vorrichtung nach Anspruch 12, wobei der Druckbehälter (2) ein Druck-Fermenter ist, in dem vorzugsweise das Gasgemisch (1) durch Fermentation aus Biomasse erzeugbar ist und wobei die flüssige Phase (3) vorzugsweise eine flüssige Fermenterphase ist.

14. Vorrichtung nach einem der Ansprüche 12 oder 13, wobei der Druckbehälter (2) über zumindest zwei Verbindungsleitungen (12, 13) derart mit der Strippingeinrichtung (4) verbunden ist, dass die flüssige Phase (3) in einem Kreislauf zwischen dem Druckbehälter (2) und der Strippingeinrichtung (4) führbar ist.

15. Vorrichtung nach einem der Ansprüche 12 bis 14, wobei die Vorrichtung zumindest eine der Strippingeinrichtung (4) nachgeschaltete Trenneinrichtung (7) aufweist, mit der ein Kohlenstoffdioxid-haltiger Stoffstrom (8) von dem Stripping-Stoffstrom (6) abtrennbar ist.

16. Vorrichtung nach Anspruch 15, wobei die Trenneinrichtung (7) eine Rektifikationskolonne, vorzugsweise eine Füllkörperkolonne und/oder eine Glockenbodenkolonne ist.

17. Vorrichtung nach einem der Ansprüche 15 oder 16, wobei die Strippingeinrichtung (4) über zumindest zwei Verbindungsleitungen (14, 15) derart mit der Trenneinrichtung (7) verbunden ist, dass das Strippingmittel (5) in einem Kreislauf zwischen der Strippingeinrichtung (4) und der Trenneinrichtung (7) führbar ist.

18. Vorrichtung nach einem der Ansprüche 15 bis 17, wobei die Vorrichtung eine Kälteeinrichtung (16) zur Verflüssigung des abgetrennten Kohlenstoffdioxid-haltigen Stoffstromes (8) bzw. des darin enthaltenen Kohlenstoffdioxids aufweist, wobei die Kälteeinrichtung (16) vorzugsweise einem oberen Ende (9) der Trenneinrichtung (7) zugeordnet ist.

## Claims

1. A method for separating a gas mixture (1) which is produced in particular by fermentation from biomass and contains at least methane and carbon dioxide, wherein the gas mixture (1) is brought into contact with a liquid phase (3) in a pressure vessel (2) in which a pressure in the range from 2 bar to 30 bar, preferably from 5 bar to 25 bar, preferably from 10 bar to 20 bar prevails, so that at least some of the carbon dioxide dissolves in the liquid phase (3), and wherein the dissolved carbon dioxide in a stripping device (4) is separated at least in part from the liquid phase (3) by at least one stripping agent (5) as part of at least one stripping mass flow (6), in particular is continuously separated, and wherein the stripping agent (5) is an alkane with two to five carbon atoms.

2. The method according to claim 1, wherein the pressure vessel (2) is a pressure fermenter in which preferably the gas mixture (1) is produced by fermentation from biomass, and wherein the liquid phase (3) is expediently a liquid fermenter phase.

3. The method according to any one of claims 1 or 2, wherein the liquid phase (3) is circulated between the pressure vessel (2) and the stripping device (4), in particular is circulated continuously.

4. The method according to any one of claims 1 to 3, wherein the stripping agent (5) is brought into contact with the liquid phase (3) in the stripping device (4) and is preferably guided in counterflow to the liquid phase (3).

5. The method according to any one of claims 1 to 4, wherein the stripping agent (5) is gaseous at least when introduced into the stripping device (4), and wherein the stripping mass flow (6) emerging from the stripping device (4) is preferably gaseous.

6. The method according to any one of claims 1 to 5, wherein the stripping agent (5) is at least one stripping agent (5) selected from the group: "ethane, propane, *n*-butane, *iso-*butane" and most preferably is iso-butane.

7. The method according to any one of claims 1 to 6, wherein the stripping mass flow (6) emerging from the stripping device (4) is fed to a separating device (7), preferably to a rectification column, wherein a carbon dioxide-containing mass flow (8) is separated from the stripping mass flow (6) in the separating device (7), in particular is separated continuously.

8. The method according to claim 7, wherein the carbon dioxide-containing mass flow (8) is removed from the separating device (7) at an upper end (9) and is preferably subsequently fed to a storage device (10).

9. The method according to any one of claims 7 or 8, wherein the carbon dioxide-containing mass flow (8) or the carbon dioxide contained therein is liquefied after removal from the separating device (7), preferably liquefied by means of a coolant.

10. The method according to any one of claims 7 to 9, wherein the stripping agent (5) accumulates in the separating device (7) as liquid stripping agent (5), preferably accumulates at a lower end (11) of the separating device (7), and is preferably returned to the stripping device (4).

11. The method according to any one of claims 1 to 10, wherein the pressure build-up in the pressure vessel (2) takes place by technical compression and/or by producing the gas mixture (1) from biomass by means of fermentation.

12. A device for separating a gas mixture (1) preferably produced from biomass by fermentation, for carrying out a method according to any one of claims 1 to 11, wherein the device has a pressure vessel (2) in which the gas mixture (1), which contains at least methane and carbon dioxide, can be brought into contact under pressure with a liquid phase (3), wherein the device has a stripping device (4) connected to the pressure vessel (2), with which carbon dioxide dissolved in the liquid phase (3) can be separated at least partially from the liquid phase (3) by at least one stripping agent (5) as part of at least one stripping mass flow (6), in particular can be separated continuously, and wherein the stripping agent (5) is an alkane with two to five carbon atoms.

13. The device according to claim 12, wherein the pressure vessel (2) is a pressure fermenter, in which preferably the gas mixture (1) can be produced by fermentation from biomass, and wherein the liquid phase (3) is preferably a liquid fermenter phase.

14. The device according to any one of claims 12 or 13, wherein the pressure vessel (2) is connected to the stripping device (4) via at least two connecting lines (12, 13) in such a way that the liquid phase (3) can be circulated between the pressure vessel (2) and the stripping device (4).

15. The device according to any one of claims 12 to 14, wherein the device comprises at least one separating device (7) downstream of the stripping device (4), with which a carbon dioxide-containing mass flow (8) can be separated from the stripping mass flow (6).

16. The device according to claim 15, wherein the separating device (7) is a rectification column, preferably a packed column and/or a bubble-cap column.

17. The device according to any one of claims 15 or 16, wherein the stripping device (4) is connected to the separating device (7) via at least two connecting lines (14, 15) in such a way that the stripping agent (5) can be circulated between the stripping device (4) and the separating device (7).

18. The device according to any one of claims 15 to 17, wherein the device comprises a cooling device (16) for liquefying the separated carbon dioxide-containing mass flow (8) or the carbon dioxide contained therein, wherein the cooling device (16) is preferably associated with an upper end (9) of the separating device (7).

## Revendications

1. Procédé de séparation d'un mélange gazeux (1), obtenu notamment par fermentation à partir de biomasse, qui contient au moins du méthane et du dioxyde de carbone, le mélange gazeux (1) étant mis en contact avec une phase liquide (3) au sein d'un récipient pressurisé (2) dans lequel la pression est comprise entre 2 bar et 30 bar, de préférence entre 5 bar et 25 bar, préférentiellement entre 10 bar et 20 bar, pour faire en sorte que le dioxyde de carbone se dissolve au moins partiellement dans la phase liquide (3), le dioxyde de carbone dissous étant au moins partiellement séparé de la phase liquide (3), notamment de manière continue, dans un organe de stripage (4) au moyen d'au moins un agent de stripage (5) en faisant partie d'au moins un flux de stripage (6), et l'agent de stripage (5) étant un alcane renfermant deux à cinq atomes de carbone.

2. Procédé selon la revendication 1, le récipient pressurisé (2) étant un fermenteur pressurisé dans lequel on obtient préférentiellement le mélange gazeux (1) par fermentation à partir de biomasse, et la phase liquide (3) correspondant de manière appropriée à une phase liquide de fermenteur.

3. Procédé selon l'une des revendications 1 à 2, dans lequel on fait passer la phase liquide (3) en circuit fermé entre le récipient pressurisé (2) et l'organe de stripage (4), notamment de manière continue.

4. Procédé selon l'une des revendications 1 à 3, l'agent de stripage (5) étant mis en contact avec la phase liquide (3) au sein de l'organe de stripage (4) alors qu'il circule, de manière préférée, à contre-courant par rapport à la phase liquide (3).

5. Procédé selon l'une des revendications 1 à 4, l'agent de stripage (5) se présentant, au moins lors de son introduction dans l'organe de stripage (4), sous une forme gazeuse, le flux de stripage (6) sortant de l'organe de stripage (4) étant également de préférence gazeux.

6. Procédé selon l'une des revendications 1 à 5, l'agent de stripage (5) étant au moins un agent de stripage (5) choisi dans le groupe : « éthane, propane, n-butane, isobutane », s'agissant avec une préférence toute particulière d'isobutane.

7. Procédé selon l'une des revendications 1 à 6, le flux de stripage (6) sortant de l'organe de stripage (4) étant introduit dans un organe de séparation (7), s'agissant préférentiellement d'une colonne de rectification, un flux contenant du dioxyde de carbone (8) étant séparé du flux de stripage (6) dans l'organe de séparation (7), ladite séparation étant notamment effectuée de manière continue.

8. Procédé selon la revendication 7, le flux contenant du dioxyde de carbone (8) étant retiré de l'organe de séparation (7) à une extrémité supérieure (9) pour ensuite être introduit, de préférence, dans un organe de stockage (10).

9. Procédé selon l'une des revendications 7 ou 8, le flux contenant du dioxyde de carbone (8) ou le dioxyde de carbone qu'il contient étant liquéfié suite à son retrait de l'organe de séparation (7), ladite liquéfaction étant préférentiellement réalisée au moyen d'un réfrigérant.

10. Procédé selon l'une des revendications 7 à 9, l'agent de stripage (5) étant recueilli sous forme d'un agent de stripage (5) liquide dans l'organe de séparation (7), le recueillement ayant préférentiellement lieu à une extrémité inférieure (11) de l'organe de séparation (7), et étant de préférence recyclé dans l'organe de stripage (4).

11. Procédé selon l'une des revendications 1 à 10, la mise sous pression du récipient pressurisé (2) étant réalisée par compression technique et/ou par génération du mélange gazeux (1) par fermentation à partir de biomasse.

12. Dispositif destiné à séparer un mélange gazeux (1), obtenu préférentiellement par fermentation à partir de biomasse, à mettre en œuvre un procédé selon l'une des revendications 1 à 11, ledit dispositif comportant un récipient pressurisé (2) dans lequel on peut mettre en contact, sous pression, le mélange gazeux (1) contenant au moins du méthane et du dioxyde de carbone avec une phase liquide (3), ledit dispositif comportant un organe de stripage (4) qui est relié au récipient pressurisé (2) et qui permet de séparer, notamment de manière continue, au moins une partie du dioxyde de carbone dissous dans la phase liquide (3) de la phase liquide (3) au moyen d'au moins un agent de stripage (5) puisqu'il fait alors partie d'au moins un flux de stripage (6), l'agent de stripage (5) étant un alcane renfermant deux à cinq atomes de carbone.

13. Dispositif selon la revendication 12, le récipient pressurisé (2) étant un fermenteur pressurisé dans lequel on obtient préférentiellement le mélange gazeux (1) par fermentation à partir de biomasse, et la phase liquide (3) étant préférentiellement une phase liquide de fermenteur.

14. Dispositif selon l'une des revendications 12 ou 13, le récipient pressurisé (2) étant relié à travers au moins deux lignes de connexion (12, 13) à l'organe de stripage (4) de manière à ce que l'on puisse faire passer la phase liquide (3) entre le récipient pressurisé (2) et l'organe de stripage (4) en circuit fermé.

15. Dispositif selon l'une des revendications 12 à 14, ledit dispositif comportant au moins un organe de séparation (7) qui est situé en aval de l'organe de stripage (4) et permet de séparer un flux contenant du dioxyde de carbone (8) du flux de stripage (6).

16. Dispositif selon la revendication 15, l'organe de stripage (7) étant une colonne de rectification, s'agissant avantageusement d'une colonne à corps de garnissage et/ou d'une colonne à plateaux à calottes.

17. Dispositif selon l'une des revendications 15 ou 16, l'organe de stripage (4) étant relié à l'organe de séparation (7) à travers au moins deux lignes de connexion (14, 15), de manière à ce que l'on puisse faire passer l'agent de stripage (5) entre l'organe de stripage (4) et l'organe de séparation (7) en circuit fermé.

18. Dispositif selon l'une des revendications 15 à 17, ledit dispositif comportant un organe de froid (16) destiné à liquéfier le flux contenant du dioxyde de carbone (8) suite à sa séparation ou le dioxyde de carbone y étant contenu, l'organe de froid (16) étant préférentiellement associé à une extrémité supérieure (9) de l'organe de séparation (7).
